# EUROPEAN PATENT SPECIFICATION

(11) **EP 2 600 796 B1**
(45) Date of publication and mention of the grant of the patent: **03.04.2019**
(21) Application number: 11814916.0
(22) Date of filing: 03.08.2011
(51) Int. Cl.: A61F 2/18, A61N 1/36, H04R 25/00, H04R 17/02, H04R 1/46

(54) **IMPLANTABLE PIEZOELECTRIC POLYMER FILM MICROPHONE**
IMPLANTIERBARES MIKROFON MIT EINER PIEZOELEKTRISCHEN POLYMERFOLIE
MICROPHONE IMPLANTABLE À FILM POLYMÈRE PIÉZOÉLECTRIQUE

(30) Priority: 03.08.2010 US 370411 P
(43) Date of publication of application: 12.06.2013
(73) Proprietor: SoundMed, LLC, Mountain View, CA 94043 (US)
(72) Inventor: PROULX, Tim, Santa Cruz, CA 95062 (US); KASSAYAN, Reza, Atherton, CA 94027 (US)
(74) Representative: J A Kemp
(86) International application number: PCT/US2011/001382
(87) International publication number: WO 2012/018400

(56) References cited:
- WO-A1-99/31933
- US-A- 4 729 366
- US-A- 5 772 575
- US-A- 5 899 847
- US-A1- 2005 113 633
- US-A1- 2009 030 529
- US-A1- 2009 043 149
- US-A1- 2009 043 149
- US-B2- 6 937 736

## Description

This application claims the benefit of priority to U.S. Prov. App. 61/370,411 filed August 3, 2010.

The present invention relates to methods and apparatuses for implantable microphones in particular microphones using piezoelectric polymer film technology, which may be used as part of hearing aid systems.

In many implantable hearing aid systems, much of, if not all of, the components of the system are positioned subcutaneously on, within or adjacent to a patient's skull, such as proximate to the mastoid process. Depending on whether some or all of the components are implanted, implantable hearing augmentation systems may be classified as either semi-implantable or fully implantable. In a semi-implantable hearing augmentation device system, one or more components of the system such as a microphone, signal processor, and transmitter may be externally located to receive, process, and inductively transmit an audio signal to implanted components such as a transducer. In a fully-implantable hearing aid system, typically all of the components, e.g., the microphone, signal processor, and transducer, are located subcutaneously. In either arrangement, an implantable transducer is utilized to stimulate a component of the patient's auditory system (e.g., tympanic membrane, ossicles and/or cochlea).

A fully implantable hearing aid system, such as those used to stimulate the tympanic membrane, the ossicles or the cochlea have inherent advantages over traditional hearing aid systems and semi-implantable hearing aid systems because a fully implantable system is completely unobservable, eliminating the appearance of a handicap; it does not occlude the ear canal, eliminating comfort/incompatibility issues and improving low frequency sound perception for those with partial hearing loss; and it allows use in environments or activities incompatible with traditional hearing aids. Enablement of a fully implantable hearing aid system requires an implantable microphone with suitable performance.

Implantable microphones described in the art for use with implantable hearing aid systems generally employ an air-conduction type electret microphone encapsulated in a biocompatible housing with a membrane that defines an air chamber. These microphones are installed subcutaneously just above and behind the ear (U.S. Pat. 6,626,822), within the bony wall of the auditory canal (U.S. Pat. 6,516,228) or at other locations in the soft tissue separated from skull-borne vibrations (U.S. Pat. 7,354,394). A thin layer of tissue covering the microphone acts as an extension of the microphone diaphragm and couples vibrations induced by external air pressure disturbances to the embedded microphone sensor. Signals detected by the microphone may be processed, amplified and sent to an implanted transducer for stimulation of the middle ear, tympanic membrane or to electrodes for stimulation of the auditory nerve.

Implantable microphones that rely on conversion of air-pressure changes within a sealed cavity to stimulate an encapsulated electret-type microphone are concerned with cavity dimensions, enclosed air pressure and membrane stiffness to provide an acceptable tradeoff between resonance frequency and sensitivity of the device. Since an implantable microphone must necessarily be hermetically sealed, with an implantable electret-type microphone, internal pressure cannot be equalized to atmosphere, so the size of the cavity affects the restoring force on the diaphragm and therefore the microphone sensitivity. Similarly, a stiff diaphragm causes a higher resonance frequency, but lower sensitivity due to the forces needed to move the membrane.

An implantable microphone using piezoelectric polymer film (U.S. Pat. 6,937,736 B2) such as polyvinylidene fluoride ("PVDF") may overcome the limitations of electret-type implantable microphones because it is well suited for detecting sound-induced vibration in tissue (whether vibration of a thin diaphragm or vibrational waves propagating through tissue) due to its high piezoelectric voltage constant, g, which relates voltage to induced strain, its low mechanical impedance, which is well matched to tissue and its general robustness and mechanical stability. Additionally, with piezoelectric polymer film, vibration is directly converted to an electrical signal by the piezoelectric effect, in contrast to existing electret-type implantable microphones that rely on conversion of mechanical vibration to pressure changes in an enclosed air cavity for subsequent detection by an air-conduction microphone.

The present invention seeks to address the limitations of electret-type (air-conducting) microphones for use in implantable hearing aids systems by providing a piezoelectric polymer film microphone that serves as an integral part of a fully implantable hearing aid system, such as a middle ear implant or cochlear implant. The piezoelectric polymer film design allows for a small package size, relative ease of construction, high durability and improved signal to noise ratio compared to implantable electret-based microphones.

WO 99/31933 discloses a microphone device claiming an increased membrane flexibility, and decreased acoustic compliance of sealed cavity. Vibrations of a membrane are transmitted through a primary air cavity, and through an aperture of a microphone.

### SUMMARY OF THE INVENTION

The present invention comprises an implantable microphone assembly according to claim 1. In one embodiment, the piezoelectric polymer film may comprise polyvinylidene fluoride ("PVDF"). In another embodiment, the piezoelectric polymer film may comprise co-polymers of PVDF such as PVDF-TrFE; PVDF-TrFE-PZT; ferroelectric polymers; piezoelectric ceramic precursors; terpolymers of vinylidene fluoride; trifluoroethylene; chlorofluoroethylene; silicon carbide (SiC)/PVDF composites. In yet another embodiment, the housing is cylindrical in shape to facilitate the anchoring of the microphone into the bone of the patient. In yet another embodiment, the piezoelectric polymer film is attached to a curved open frame structure such that the film serves as a diaphragm and seals one end of the housing. A thin biocompatible protective layer can be disposed on the surface of the film to be in contact with the tissue.

In another example, not in accordance with the invention, a piezoelectric polymer film microphone uses a non-curved (i.e., flat) open frame structure. In this case, the spherically pre-formed piezoelectric polymer film is self supported and is attached around its perimeter to the frame. The curvature may be directed toward the tissue to present a convex surface, or preferably (due to mechanical stability when loaded with tissue) a concave surface. In the case of a concave surface, the depression is filled with a cast silicone rubber contact layer to provide a flat or slightly convex tissue-contact surface. A self supported cylindrical sensor may alternatively be created by clamping/bonding the edges of the film (in the 1-direction) but leaving the sides free. Curvature in the edge-supported cylindrical film may be induced by pre-forming the film or by
casting/bonding a cylindrically-curved silicone rubber layer onto its surface to present a flat or slightly convex tissue-contact surface.

A piezoelectric polymer film microphone can incorporate a film wrapped around a silicone rubber contact pad in which a normal force on the pad generates a tension in the film axis due to the radial expansion of the rubber pad. The rubber contact pad incorporates a cylindrical section that is clamped against a stiff platform incorporated into the housing. The piezoelectric polymer film is wrapped around the cylinder and bonded to itself with an epoxy or cyanoacrylate or other adhesive.

A curved piezoelectric polymer film surface can be created using a solid curved frame with ridges that support the film and create thin air gaps between the film and frame. Small holes in the frame couple the air gaps with the air cavity behind the plate to reduce stiffness of the system. This arrangement may provide improved mechanical stability and reduce the effect of low frequency vibrations traveling within the tissue, such as those caused by user movements or breathing. It also provides additional microphone design flexibility, in that hole sizes and spacing and size of supporting ridges can be adjusted to fine tune the response.

The implantable piezoelectric polymer film microphone (including, but not limited to, the PVDF microphone) may be subcutaneously implanted in the bony or cartilaginous wall of the ear canal, disposed on the surface or implanted in the temporal bone on the posterior or anterior side of the ear (mastoid region), or in any soft tissue in a region that facilitates the reception of acoustic signals. The implantable piezoelectric polymer film microphone may be anchored into the posterior bony wall of the ear canal. This allows the microphone to take advantage of the natural sound amplification provided by the ear geometry, and makes implantation easier because of the thin dermis layer in this anatomical region. Additionally, this mounting may protect the piezoelectric polymer film microphone from mechanical damage. If mounted to the bone of the skull, the implantable piezoelectric polymer film microphone of the present invention may incorporate a rubber spacer to reduce the effect of bone-conducted vibrations caused by the user's speech.
Fig. 1 shows an example of how a curvature translates normally directed pressure into tensile stresses along the film axis that can be much larger than the applied stress.
Fig. 2 shows a piezoelectric film microphone incorporating a film wrapped around a deformable pad, such as a silicone or other rubber contact pad in which a normal force on the pad generates a tension in the film axis due to the radial expansion of the rubber pad.
Fig. 3 shows a piezoelectric film with the stretch direction (1-direction) indicated, where the edges of the film (in the 1-direction) are clamped but the sides are not.
Fig. 4 shows the implantable piezoelectric polymer film microphone of the present invention implanted in the temporal bone on the posterior or anterior side of the ear (mastoid region).
Figs. 5A and 5B show cross-sectional side and top views, respectively, of one embodiment of a microphone comprising a curved frame defining a circular opening which supports a piezoelectric polymer film.
Figs. 6A and 6B show cross-sectional side and top views, respectively, of another embodiment of a microphone comprising a curved frame defining a rectangular opening which supports a piezoelectric polymer film.
Figs. 7A and 7B show cross-sectional side and top views, respectively, of another embodiment of a microphone comprising a non-curved frame with a pre-formed piezoelectric polymer film that is self supported and incorporates a silicone rubber tissue contact lens. The silicone rubber lens induces a curvature in the piezoelectric polymer film.
Figs. 8A and 8B show cross-sectional side and top views, respectively, of another embodiment of a microphone comprising a non-curved frame defining a rectangular opening with a silicone rubber lens which defines a curvature for the piezoelectric polymer film.
Figs 9A and 9B show cross-sectional side and top views, respectively, of another embodiment of a microphone having a curved frame with ridges that support a piezoelectric polymer film and create thin air gaps between the film and frame. The air gaps couple to an air cavity behind the frame via small holes in the frame.
Figs. 10A and 10B show anterior and side views, respectively, of an example of the microphone implanted in subcutaneous tissue within, e.g., the neck, torso, etc. beneath the skin.
Fig. 11 shows a cross-sectional side view of the microphone of Fig. 8 implanted subcutaneously in the bony wall of the ear canal.
Fig. 12 shows a cross-sectional side view of the microphone assembly of Fig. 2 implanted subcutaneously in the bony wall of the ear canal.
Figs. 13A and 13B show cross-sectional anterior and side views, respectively, of another example of the microphone of Figs. 5 and 6 implanted in the bony wall of the ear canal.
Figs. 14A and 14B show cross-sectional view of microphone having a housing that enables the implantation of the microphone within or along the skull in proximity to the patient's outer ear, e.g., posterior or anteriorly of the outer ear.

The piezoelectric polymer film microphone can be implanted in suitable sites of the body by surgical techniques that are used for the implantation of electret-type microphones, which are well known to those of skill in the art. The piezoelectric polymer microphone of the present invention may be subcutaneously implanted in the bony or cartilaginous wall of the ear canal (i.e., the bony wall of the ear canal), disposed on the surface or mounted to the temporal bone on the posterior or anterior side of the ear (mastoid region), or in any soft tissue in a region that facilitates the reception of acoustic signals such as in the soft tissue of the neck, or in other locations as described in U.S. Pats. 6,626,822, 6,516,228 and 7,354,394. The microphone may be anchored into the posterior bony wall of the ear canal to take advantage of the natural sound amplification provided by the ear geometry, and because of the thin dermis layer in this area making implantation easier. Additionally, this mounting may protect the microphone from mechanical damage. If mounted to the bone of the skull (i.e., the mastoid bone) the piezoelectric polymer film microphone of the present invention may incorporate a rubber spacer to reduce the effect of bone-conducted vibrations caused by user speech.

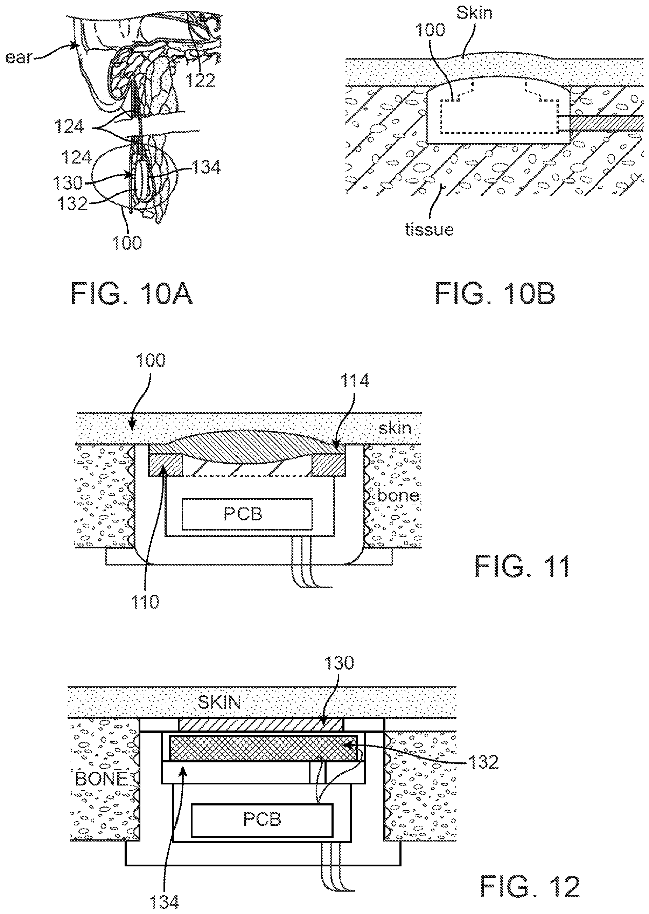

To reduce size and enable a secure attachment to the bony wall of the ear canal or to the mastoid by osteointegration, a housing (such as a cylindrical housing) having screw-type threads or groove features for engagement with the bone and to facilitate insertion therein may be employed. The threads may extend over the majority of the housing length (such as for full insertion into the bony wall), or on a distal portion of the housing (such as for partial insertion into the bone of the skull). The housing may be machined or manufactured from titanium or other biocompatible metals known in the art, such as stainless steel or gold, or from any type of implant-grade plastic known in the art such as PEEK. A plastic housing incorporating a conductive paint or metal plating on its interior may be used to reduce susceptibility of the microphone to electromagnetic interference. The housing may incorporate a distal flange that improves mechanical positioning and anchoring in place, especially in those areas of the skull in which the housing is positioned adjacent to an air void or cavity, such as that shown in FIG. 11. The housing may be other shapes than cylindrical, such as oval or rectangular, depending on the implant location or method of attachment, as one skilled in the art may readily discern.

In one embodiment, the piezoelectric polymer film microphone sensor of the present invention is constructed by bonding (e.g., with cyanoacrylate, epoxy or double-sided adhesive) or, in the case of PVDF film, by mechanically clamping a pre-formed spherically shaped PVDF film (e.g., 5mm diameter) to a spherically curved and open titanium frame. In the case in which the piezoelectric polymer film microphone of the present invention is comprised of PVDF film, the PVDF film is pre-formed by stretching it over a steel sphere at elevated temperature (e.g., 80°C), under a poling field of 40V/micron (R. Lerch, "Electroacoustic transducers using piezoelectric polyvinylidene fluoride films", J. Acoust. Soc. Amer., vol. 66, no. 4, pp. 952-954, 1979. Alternatively, pre-forming can be avoided by attaching a rectangular layer of PVDF film (e.g., 5x5mm, 100 micron thick) to a curved and open frame such that the stretch direction (known as the "1" direction) of the film is along the radius of curvature of the frame (U.S. Pat. 6,937,736). Other piezoelectric polymer films such as copolymers of PVDF (e.g., PVDF-TrFE; PVDF-TrFE-PZT; ferroelectric polymers; piezoelectric ceramic precursors; terpolymers of vinylidene fluoride; trifluoroethylene; chlorofluoroethylene; silicon carbide (SiC)/PVDF composites; etc.) also may be used and are contemplated as part of the present invention.

In one embodiment, the frame is machined from a biocompatible metal, such as 304 or 316 stainless steel or titanium. To minimize the amount of inactive film material (which adds to parasitic capacitance), the width of the frame edge is maintained at a practical minimum to effectively clamp the film and resist deflection. A width of about 1mm may be used. Radius of curvature directly impacts microphone sensitivity and resonance frequency (due to the effect on film compliance). A frame radius of, e.g., 10mm - 25mm, may be used to provide a resonance frequency above the primary speech frequency band (e.g., 300 - 4kHz) while maintaining sufficient device sensitivity.

In one embodiment, signal conditioning circuitry is positioned as close as possible to the sensor to drive further electrical stages or electrical leads. The pre-amplifier incorporates a high input impedance (e.g., >10M Ohm) JFET transistor for impedance conversion and signal gain and is packaged with the sensor in the microphone housing. The JFET amplifier has lower electronic noise than typical MOSFET amplifiers used for electret-based microphones. High pass filtering may be employed after signal amplification to reduce electronic noise below, e.g., 100Hz. Depending on the distance between the microphone sensor and the hearing device control unit, and at the expense of sensitivity, the pre-amplifier may alternatively be located in the control unit, further simplifying the microphone design and reducing overall size.

In one embodiment, the PVDF film sensor includes a termination board or termination pads that allow attachment to the enclosed pre-amplifier by mechanical means or by conductive epoxy (e.g. E-Solder®, Von Roll Isola). The conditioned output signals are connected to the exterior of the housing by means of small lead through connector hermetically sealed into the housing. A thin, flexible shielded cable or individual (twisted) insulated wires connect the microphone to the battery/control unit. The electrical termination scheme may alternately utilize lithographically formed wires in a thin laminate for connection to hermetically sealed lead-throughs as described in U.S. Pat. 6,516,228 incorporated herein by reference.

In another embodiment, the frame with attached PVDF diaphragm is integrated into the microphone housing by mechanical fasteners or adhesives creating a hermetic seal. To protect the exposed PVDF electrode surface, a conformal layer of biocompatible polymer (e.g., 50 microns of parylene C) is vapor deposited onto the sensor to create a contact layer. The polymer provides a good match between the PVDF and the tissue. Alternate contact layer materials include polyimide or polyester laminates that may be incorporated into the film during its fabrication or applied by adhesives during microphone construction, or a thin layer of implant grade silicone rubber (e.g., Applied Silicone LSR30) cast onto the microphone diaphragm surface. To minimize mechanical loading effects and to reduce the microphone profile, the contact layer may be limited to 0.5mm thickness.

In an alternate arrangement, a piezoelectric polymer film sensor uses a non-curved (i.e., flat) frame structure, e.g., an open frame structure. In this particular arrangement, the spherically pre-formed PVDF film is self supported and is attached around its perimeter to the frame. The curvature may be directed toward the tissue to present a convex surface, or preferably (due to mechanical stability when loaded with tissue) a concave surface. In the case of a concave surface, the depression is filled with a cast silicone rubber contact layer to provide a flat or slightly convex tissue-contact surface. In an alternate embodiment, a self supported cylindrical sensor may be created by clamping/bonding the edges of the film (in the 1 -direction) but leaving the sides free. Curvature in the edge-supported cylindrical film may be induced by pre-forming the film or by casting/bonding a cylindrically-curved silicone rubber layer onto its surface to present a flat or slightly convex tissue-contact surface.

In yet a further embodiment, a curved piezoelectric polymer film surface is created using a solid curved frame with ridges that support the film and create thin air gaps between the film and frame. Small holes in the frame couple the air gaps with the air cavity behind the plate (R. Lerch, G.M. Sessler, "Microphones with rigidly supported piezopolymer membranes", J. Acoust. Soc. Amer., vol. 67, no. 4, pp. 1379-1381, 1980) to reduce stiffness of the system. This embodiment is designed to provide improved mechanical stability and reduce the effect of low frequency vibrations traveling within the tissue, such as those caused by user movements or breathing. It also provides additional microphone design flexibility, in that hole sizes and spacing and size of supporting ridges can be adjusted to fine tune the response. In an alternate embodiment, a piezoelectric polymer film tissue contact microphone incorporates a film wrapped around a silicone rubber contact pad in which a normal force on the pad generates a tension in the film axis due to the radial expansion of the rubber pad. The rubber contact pad incorporates a cylindrical section that is clamped against a stiff platform incorporated into the housing. The piezoelectric polymer film is wrapped around the cylinder and bonded to itself with an epoxy or cyanoacrylate or other adhesive. A small exposed tab allows access to the bottom electrode. Electrical leads are attached to both top and bottom electrodes and routed through holes in the platform to the microphone enclosure for signal conditioning and amplification.

Piezoelectric film such as PVDF is well suited for use as an implantable tissue contact sensor due to its high piezoelectric voltage constant, g, which relates voltage to induced strain, its low mechanical impedance, which is well matched to tissue and its general robustness and mechanical stability. Additionally, with piezoelectric film, tissue vibration is directly converted to an electrical signal by the piezoelectric effect, in contrast to contact sensors that rely on conversion of mechanical vibration to pressure changes in an enclosed air cavity for subsequent detection by an air-conduction microphone (such as those described in U.S. Pats. 6,516,228 and 7,433,484).

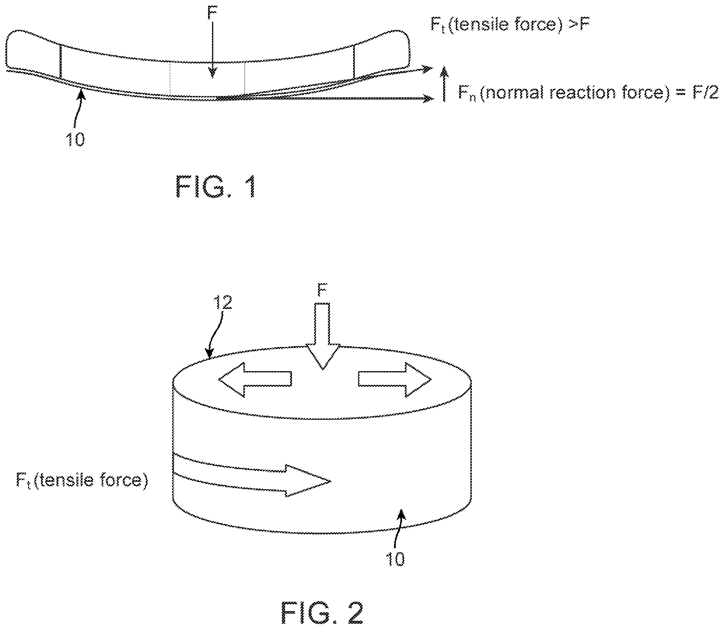

When clamped to a curved open frame structure, a piezoelectric polymer film **10,** such as a PVDF film, provides very high sensitivity to normally directed mechanical displacement and its frequency response is flat when operated below resonance. The curvature translates a normally directed pressure or force **F** into tensile stresses along the film axis that can be much larger than the applied stress (FIG 1). The induced film strain generates charge on the film electrodes in proportion to the applied pressure. Film thickness, radius of curvature (ROC) and electrode area may be adjusted to affect electrical impedance, sensitivity, resonance frequency and mechanical impedance, thus allowing fine tuning to the application. FIG. 2 illustrates an example where a normally directed force **F** may be applied to a curved structure **12,** which induces radial expansion over the curved structure and generates a tensile force in the circumferentially bonded film.

The microphone sensor can be constructed by bonding (e.g., with cyanoacrylate, epoxy or double-sided adhesive) or mechanically clamping a layer of piezoelectric polymer film such as PVDF film (e.g. 10mm x 20mm, 52 micron thick) to a curved and open metal frame such that the stretch direction (known as the "1" direction) of the film is along the radius of curvature of the frame (U.S. Pat. 6,937,736 incorporated herein by reference). Other piezoelectric films such as copolymers of PVDF (e.g., PVDF-TrFE) may also be used.

The frame may be constructed of a biocompatible metal, such as 304 or 316 stainless steel or titanium. To minimize the amount of inactive film material (which adds to parasitic capacitance), the width of the frame edge is maintained at a practical minimum to effectively clamp the film and resist deflection. A width of, e.g., 1-2mm, may be used in one example. Radius of curvature directly impacts microphone sensitivity and resonance frequency (due to the effect on film compliance). A frame radius of, e.g., 5mm - 20mm, may be used to provide a resonance frequency above the primary speech frequency band (e.g., 300 - 4kHz) while maintaining sufficient device sensitivity. The frame is integrated into the microphone housing, e.g., by mechanical fasteners or adhesives. Moreover, the frame may be configured in a number of different shapes, elliptical, circular, etc. depending upon the desired characteristics. Additionally, in alternative variations, the frame may be omitted from the enclosure and/or the piezoelectric polymer film may be secured directly to the housing and unsupported by the frame while the piezoelectric polymer film remains adhered to and in vibrational contact with the contact surface of the enclosure.

A contact layer (lens) of silicone RTV or polyurethane rubber (e.g., NuSil Med-6015 or Dow Corning X3-6121) is cast in place on the piezoelectric polymer film **10.** The lens casting process ensures intimate mechanical contact between the lens and piezoelectric polymer film (such as PVDF film) over the entire surface and acts to seal the front surface of the microphone assembly from liquid intrusion. An alternate approach is to attach a piezoelectric polymer film to a pre-molded rubber contact layer using a flexible adhesive. This requires care to ensure intimate contact over the active film surface and a water-tight seal at the lens/housing interface. To minimize mechanical loading effects and to reduce the microphone profile, the contact lens may be limited, e.g., to 1-2mm in thickness.

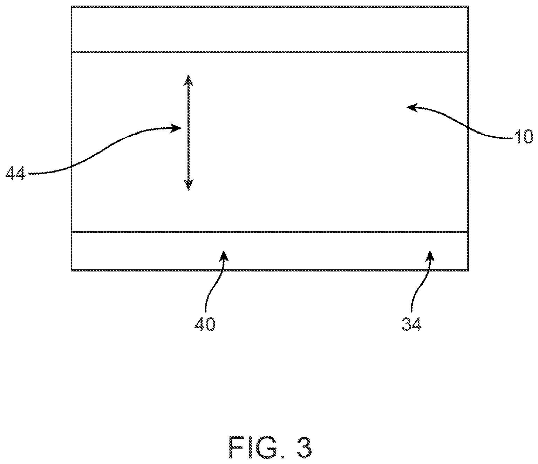

FIG 3 shows an alternate arrangement for a piezoelectric film **10** uses a flat open frame **34** where the first set of edges of the film opposite to one another (in the 1-direction **44**) are clamped **40** but the opposing second set of sides are not. Static (i.e., "DC") pressure on the contact lens **40** (such as when installed against the tissue) causes the film to deflect from a straightened or flattened neutral position, resulting in the curved configuration described above. Here, the amount of induced curvature is defined by the DC force applied, thus sensitivity and frequency response of the sensor will vary during use. However, this arrangement may result in a light/smaller device and simplified construction.

With this architecture, the amount of film curvature may be alternatively adjusted/controlled electronically by applying a DC electric field by means of a DC boost converter circuit connected via leads to first and second electrodes.

Alternately, the desired piezoelectric film curvature may be achieved by adhering the film to a rubber contact layer having a pre-defined curvature using a flexible adhesive and clamping the edges (in the 1-direction) between the frame and housing.

As with the curved/clamped film arrangement described earlier, the tensile force acts on the edge of the film; the small effective area of the film edge causes a much higher stress than that measured at the surface of the film, resulting in higher voltage for the same incoming pressure.

The high capacitance of the piezoelectric polymer film (such as the PVDF film) or electret microphone sensor calls for signal conditioning circuitry positioned as close as possible to the sensor in order to effectively drive further electrical stages. The pre-amplifier may incorporate a high input impedance (e.g., >10M Ohm) low noise JFET transistor or commercial electret amplifier chip for impedance conversion and signal gain and may be packaged with the sensor in the microphone housing. Band pass filtering may be employed after signal amplification to emphasize the speech frequency range, such as 300 Hz - 4000 Hz.

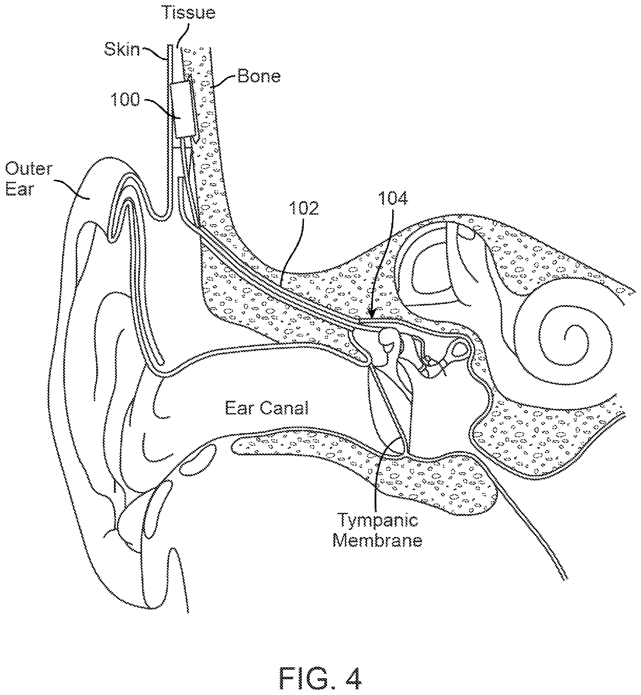

An example of how the piezoelectric polymer microphone may be placed is illustrated in FIG 4 which shows microphone **100** implanted under the skin of a patient. Microphone **100** may be implanted (either within the subcutaneous tissue, bone, or both) behind or above one or both of the patient's ear(s) in this example with one or more wires **102** electrically coupling the microphone to the bones **104** of the inner ear. The microphone **100** may be positioned just under the skin to receive vibrational waves through the skin such that the microphone **100** may receive the vibrations, as described above. The processed acoustic vibrations may be electronically transmitted through the one or more wires **102** to stimulate the bones **104** of the inner ear to provide the patient the sensation of hearing. The distal end of the one or more wires **102** may be configured to contact and/or be secured to the bones **104** of the inner ear or to tissue in vibrational contact with the bones **104.**

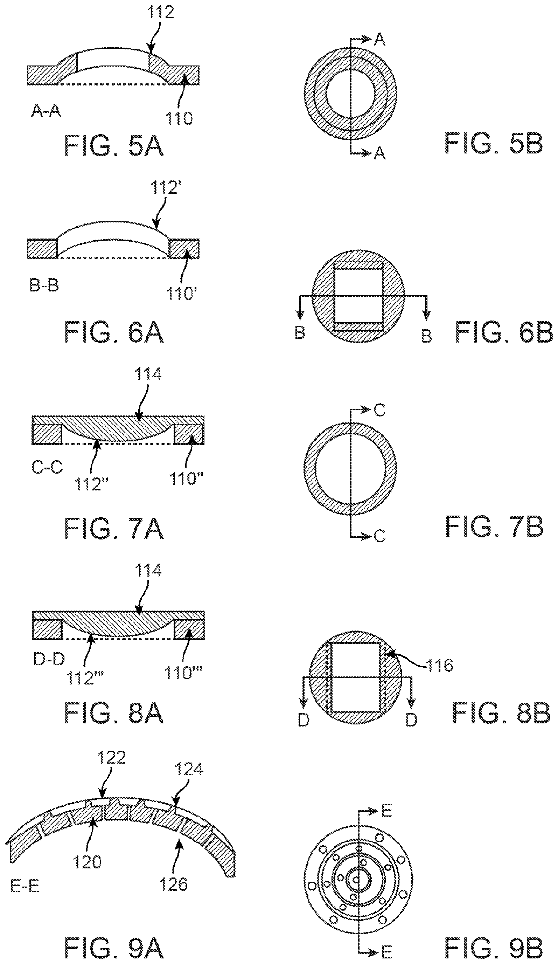

As described above, the frame and piezoelectric polymer film (e.g., PVDF film) contained within the housing of the microphone may be configured in a number of different shapes. FIGS 5A and 5B illustrate partial cross-sectional side and top views of one example where frame **110** may be configured into a circular configuration having a curved shape. Film **112** may also be circularly configured and positioned upon the frame **110,** as shown. FIGS 6A and 6B illustrate cross-sectional side and top views of another variation where the frame **110'** may be similarly shaped into a circular configuration while the film **112'** may be configured into a square or rectangular configuration supported upon the frame **110'** either along two opposed edges of the film **112'** or around the entire periphery of the film **112'.**

FIGS 7A and 7B illustrate cross-sectional side and top views of another variation of the frame **110"** which is non-curved into a circular configuration and with film **112"** supported around the periphery of the frame **110".** However, in this example, an additional layer of silicone rubber forming a silicone lens **114** may be placed atop the film **112"** such that the film **112"** is sandwiched between the frame **110"** and silicone lens **114.** FIGS 8A and 8B illustrate a similar variation where the film **112'"** may be shaped into a square or rectangular configuration upon the circular frame **110"'** which is non-curved with the silicone lens **114** layered atop the film **112"'** inducing a curvature in the film. However, the film **112'"** may be supported along opposite edges or along the entire periphery of the film **112"'** such that the film **112'"** is formed into a curved shape formed by the silicone lens **114.**

FIGS 9A and 9B illustrate partial cross-sectional side and top views of yet another variation where a film **122** may be supported via a plurality of protruding supports such as ridges **124** projecting from a circular frame **120.** The frame **120** may optionally define one or more openings or holes **126** which allow for communication between the film **122** and an internal air cavity behind the frame.

FIGS 10A and 10B illustrate another variation for implanting the microphone in alternative locations on the body. In this example, the microphone **100** is shown implanted directly into the subcutaneous tissue of the neck below and/or behind the patient's ear rather than secured or anchored within a bone structure. Other soft tissue regions of the body may also be utilized for implantation of the microphone, such as the torso, etc.

FIG 11 illustrates a cross-sectional side view of another example of the microphone **100** (such as the microphone assembly of FIG 8) implanted directly within a bone, such as bony wall of the ear canal in which the assembly is anchored, such that the silicone lens **114,** formed to have a flattened or curved configuration with a thickness of about, e.g., 0.3 mm to 0.5 mm, may impart its curvature to the film **112** positioned over a lower surface of the silicone lens **114,** as previously described. The thickness of the silicone lens **114** may be minimized to limit the mechanical loading on the film **112.** The silicone lens **114** may have an upper curved surface which contacts the subcutaneous tissue or skin while both the silicone lens **114** and film **112** are supported by the frame **110.** The entire assembly of the silicone lens **114,** film **112,** and frame **110** may be supported by the implant housing **123** secured to the bone and the film **112** may be in electrical communication with an electronics assembly **121** positioned along, e.g., a printed circuit board, secured within the implant **123.** The implant housing **123** may be optionally threaded for facilitating the anchoring into the bone. The electronics assembly **121** may in turn be electrically coupled to the bones of the inner ear, as previously described.

FIG 12 illustrates a cross-sectional side view of yet another example of a microphone assembly which may be implanted within a bone such that a silicone or other rubber pad **130** may be in contact with the subcutaneous tissue of skin for receiving auditory vibrations. The pad **130** may have a circumferentially oriented piezoelectric polymer film such as PVDF film **132** supported by the implant housing **123** while positioned upon a relatively stiff platform **134.** As described herein, as the auditory vibrations are conducted through skin and received by the pad **130,** the corresponding changes in size imparted by the pad 130 into the film **132** may be received and processed by the electronics assembly **121** for further electronic transmission into the body.

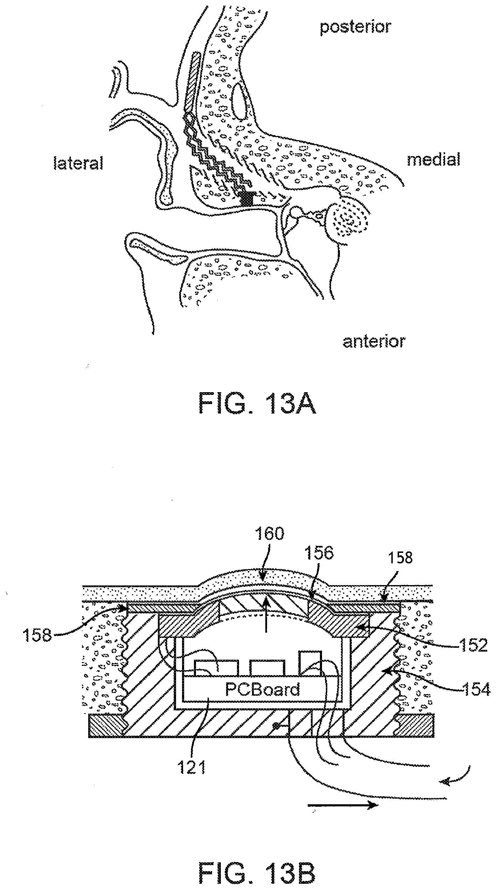

FIGS 13A and 13B illustrate the invention of how the microphone assembly may be implanted into the patient's body. In this example, the circular frame **152** supports the piezoelectric polymer film **156** such that the film **156** has a curvature (e.g., a radius of curvature of 20 mm to 25 mm) which contacts the subcutaneous tissue or skin **160.** The film **156** and/or implant housing **154** (e.g., having a diameter of 8 mm to 10 mm) may be optionally coated with a biocompatible coating, e.g., parylene, which seals the assembly. The implant housing **154** may be secured directly into a bone, such as the skull, via threaded features along the housing or into the tissue underlying the skin **160** (in which case the housing may be at least partially unthreaded for that portion which embeds into the soft tissue) and may further support the frame **152,** film **156,** and a retaining ring **158** which secures the assembly within the housing **154.** The housing **154** may further include one or more flanges, as shown, along a portion of the housing structure which enables the microphone assembly to be anchored or secured at anatomical sites having one or more voids, such as air cavities or air pockets as are commonly found within the bones of the skull or other bony regions of the body. The film **156** may be electrically coupled to an electronics assembly **121** which may be further electrically coupled to the structures of the inner ear via one or more electrical leads or cables which some or all may be shielded, as described herein.

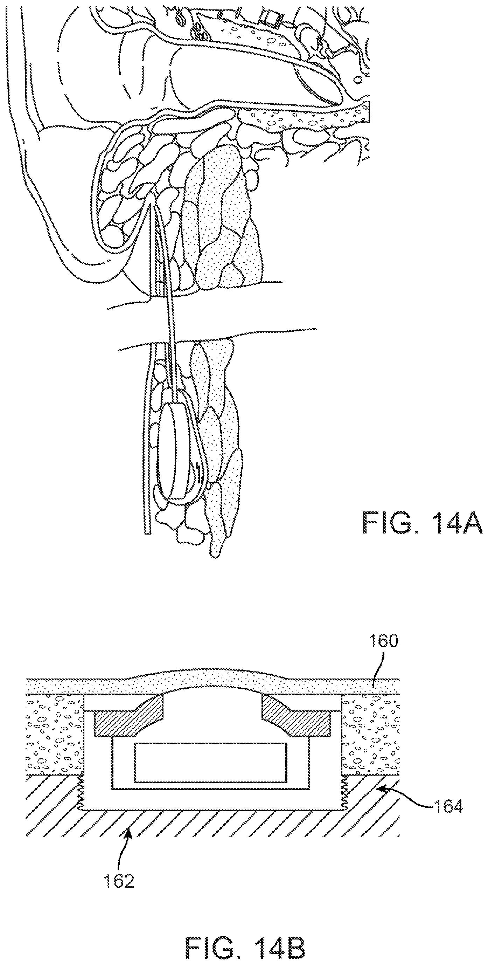

FIG 14A and 14B illustrate yet another example of how the microphone assembly **162** may be threaded just partially into the bone **164** while remaining above the bone

surface and within the subcutaneous tissue and in contact with the underlying surface of skin **160.**

Each of the microphone assemblies disclosed herein and as shown in FIGS. 2 and 5-9 may be incorporated with any of the housings disclosed herein and as shown in FIGS. 11, 12, 13B, and 14B such that the microphone assemblies are anchored or secured in bone, in soft tissue, in a combination of bone and soft tissue, or in a combination of bone adjacent to soft tissue and/or adjacent to a void (such as an air cavity or air pocket). One of skill in the art is capable of matching a particular microphone assembly with a suitable housing such that the implantable microphone device (i.e., microphone assembly and housing) is configured appropriately for the chosen anatomical site of implantation.

Due to size constraints of the microphone itself, the components of the microphone assembly may be separated from one another while remaining in electrical communication. A first assembly, e.g., the microphone, may be separated from a second assembly such as an opposing side of the assembly which may incorporate additional digital signal processing electronics, transmitter or receiver circuitry (or both), an antenna and battery (e.g., lithium ion), depending on the application. Charging may be accomplished using inductive means (in which an induction coil is required in the appliance package) or by direct coupling of exposed electrical contacts.

The implantable piezoelectric polymer film microphone of the present invention may be used as an integral part of a hearing system, such as a middle-ear or cochlear implant. The signals detected by the implantable microphone may be processed/filtered, amplified and wirelessly transmitted using, e.g., near field magnetic induction (NFMI) or low-power radio frequency (RF) link to an implanted receiving coil and sent to the implanted hearing device control module for further signal processing and stimulation of the middle ear or auditory nerve.

## Claims

1. An implantable microphone assembly (100), comprising:
a housing (123, 154) having a diameter of 8 mm to 10 mm which is configured for subcutaneous implantation within a patient;
a circular frame (110, 152) defining a central opening (126) and having a curvature and positioned within the housing;
a piezoelectric polymer film (112, 122) secured to the frame and where the housing is configured to position the frame along an upper portion of the housing and where the frame has a radius of curvature of 20 mm to 25 mm such that the film presents a convex surface configured to be in direct vibrational contact with subcutaneous tissue;
an electronics assembly (121) in electrical communication with the film; and
one or more wires (102) connected to the electronics assembly at a proximal end and further having a distal end configured for coupling to bones of an inner ear of the patient.

2. The assembly of claim 1 wherein the piezoelectric polymer film is chosen from: PVDF and copolymers of PVDF.

3. The assembly of claim 1 wherein the piezoelectric polymer film comprises PVDF.

4. The assembly of claim 1 wherein the housing is threaded for securement to a bone of the patient.

5. The assembly of claim 1 wherein the frame comprises a curved surface.

6. The assembly of claim 1 wherein the piezoelectric polymer film (112, 122) is supported via a plurality of protruding supports (124) projecting from the circular frame.

7. A method of detecting an auditory signal via an implantable microphone assembly (100) comprising a housing (123, 154) having a diameter of 8 mm to 10mm positioned beneath a region of skin subcutaneously within a patient such that a piezoelectric polymer film (112, 122) secured to a circular frame (110) defining a central opening (126) and having a curvature and positioned within the housing is in vibrational contact with the region of tissue, where the housing is configured to position the frame along an upper portion of the housing and where the frame has a radius of curvature of 20 mm to 25 mm such that the film presents a convex surface configured to be in direct vibrational contact against subcutaneous tissue, the method comprising:
receiving an auditory signal vibrationally transmitted through the region of skin and against the film;
actuating the film via the auditory signal such that an electric signal representative of the auditory signal is produced by the film; and
transmitting the electric signal to an inner ear of the patient.

8. The method of claim 7 wherein the piezoelectric polymer film comprises PVDF.

9. The method of claim 7 wherein actuating the film further comprises processing the electric signal via an electronics assembly (121) positioned within the housing.

10. The method of claim 7 wherein actuating the film comprises imparting an expansion or contraction to a pad (130) secured within the housing and in contact with the film.

11. The method of claim 7 wherein transmitting the electric signal comprises transmitting the signal via one or more wires (102) attached to the inner ear.

12. The method of claim 7 wherein receiving an auditory signal comprises receiving the signal via a tissue contact portion of the housing which has an impedance matched to the region of tissue.

13. The method of claim 7 wherein the piezoelectric polymer film (112, 122) is supported via a plurality of protruding supports (124) projecting from the circular frame.

## Patentansprüche

1. Implantierbare Mikrofonanordnung (100), umfassend:
ein Gehäuse (123, 154), das einen Durchmesser von 8 mm bis 10 mm hat, das zur subkutanen Implantation in einem Patienten konfiguriert ist;
einen kreisförmigen Rahmen (110, 152), der eine zentrale Öffnung (126) definiert und eine Krümmung hat und in dem Gehäuse positioniert ist;
einen piezoelektrischen Polymerfilm (112, 122), der an dem Rahmen gesichert ist und wo das Gehäuse konfiguriert ist, um den Rahmen entlang eines oberen Abschnitts des Gehäuses zu positionieren und wo der Rahmen einen Krümmungsradius von 20 mm bis 25 mm hat, sodass der Film eine konvexe Oberfläche darstellt, die konfiguriert ist, um in unmittelbarem Vibrationskontakt mit subkutanem Gewebe zu sein;
eine Elektronik (121) in elektrischer Verbindung mit dem Film; und
ein oder mehrere Kabel (102), die mit der Elektronik an einem proximalen Ende verbunden sind und ferner ein distales Ende zum Koppeln an Knochen eines Innenohrs des Patienten konfiguriert haben.

2. Anordnung nach Anspruch 1, wobei der piezoelektrische Polymerfilm ausgewählt ist aus: PVDF und Copolymeren von PVDF.

3. Anordnung nach Anspruch 1, wobei der piezoelektrische Polymerfilm PVDF umfasst.

4. Anordnung nach Anspruch 1, wobei das Gehäuse zur Befestigung an einen Knochen des Patienten mit Gewinden versehen ist.

5. Anordnung nach Anspruch 1, wobei der Rahmen eine gekrümmte Oberfläche umfasst.

6. Anordnung nach Anspruch 1, wobei der piezoelektrische Polymerfilm (112, 122) über eine Vielzahl von vorstehenden Trägern (124) getragen wird, die von dem kreisförmigen Rahmen vorstehen.

7. Verfahren des Erfassens eines akustischen Signals über eine implantierbare Mikrofonanordnung (100), umfassend ein Gehäuse (123, 154), das einen Durchmesser von 8 mm bis 10 mm hat, das unter einem Hautbereich subkutan in einem Patienten positioniert ist, sodass ein piezoelektrischer Polymerfilm (112,122), der an einem kreisförmigen Rahmen (110) befestigt ist, der eine zentrale Öffnung (126) definiert und eine Krümmung hat und in dem Gehäuse positioniert ist, in Vibrationskontakt mit dem Bereich des Gewebes ist, wo das Gehäuse konfiguriert ist, um den Rahmen entlang eines oberen Abschnitts des Gehäuses zu positionieren und wo der Rahmen einen Krümmungsradius von 20 mm bis 25 mm hat, sodass der Film eine konvexe Oberfläche darstellt, die konfiguriert ist, um in unmittelbarem Vibrationskontakt mit subkutanem Gewebe zu sein, wobei das Verfahren Folgendes umfasst:
Empfangen eines akustischen Signals, das durch den Hautbereich und gegen den Film schwingend übertragen wird;
Betätigen des Films über das akustische Signal, sodass ein elektrisches Signal, das das akustische Signal darstellt, durch den Film hergestellt wird; und
Übertragen des elektrischen Signals an ein Innenohr des Patienten.

8. Verfahren nach Anspruch 7, wobei der piezoelektrische Polymerfilm PVDF umfasst.

9. Verfahren nach Anspruch 7, wobei das Betätigen des Films ferner das Verarbeiten des elektrischen Signals über eine Elektronik (121) umfasst, die in dem Gehäuse positioniert ist.

10. Verfahren nach Anspruch 7, wobei das Betätigen des Films das Verleihen einer Ausdehnung oder eines Zusammenziehens an ein (130) Kissen umfasst, das in dem Gehäuse und in Kontakt mit dem Film befestigt ist.

11. Verfahren nach Anspruch 7, wobei das Übertragen des elektrischen Signals das Übertragen des Signals über ein oder mehrere Kabel (102) umfasst, die an dem Innenohr befestigt sind.

12. Verfahren nach Anspruch 7, wobei das Empfangen eines akustischen Signals das Empfangen des Signals über einen Gewebekontaktabschnitt des Gehäuses umfasst, das eine Impedanz hat, die dem Bereich des Gewebes angepasst ist.

13. Verfahren nach Anspruch 7, wobei der piezoelektrische Polymerfilm (112, 122) über eine Vielzahl von vorstehenden Trägern (124) getragen wird, die von dem kreisförmigen Rahmen vorstehen.

## Revendications

1. Ensemble microphone implantable (100), comprenant :
un boîtier (123, 154) ayant un diamètre de 8 mm à 10 mm qui est configuré pour l'implantation sous-cutanée chez un patient ;
un cadre circulaire (110, 152) définissant une ouverture centrale (126) et ayant une courbure et étant positionné à l'intérieur du boîtier ;
un film polymère piézoélectrique (112, 122) fixé au cadre et où le boîtier est configuré pour positionner le cadre le long d'une portion supérieure du boîtier et où le cadre a un rayon de courbure de 20 mm à 25 mm de telle sorte que le film présente une surface convexe configurée pour être en contact vibratoire direct avec un tissu sous-cutané ;
un ensemble électronique (121) en communication électrique avec le film ; et
un ou plusieurs fils (102) connectés à l'ensemble électronique à une extrémité proximale et en outre ayant une extrémité distale configurée pour se coupler à des os d'une oreille interne du patient.

2. Ensemble selon la revendication 1, dans lequel le film polymère piézoélectrique est choisi à partir de : PVDF et copolymères de PVDF.

3. Ensemble selon la revendication 1, dans lequel le film polymère piézoélectrique comprend du PVDF.

4. Ensemble selon la revendication 1, dans lequel le boîtier est fileté pour la fixation à un os du patient.

5. Ensemble selon la revendication 1, dans lequel le cadre comprend une surface courbée.

6. Ensemble selon la revendication 1, dans lequel le film polymère piézoélectrique (112, 122) est supporté par l'intermédiaire d'une pluralité de supports saillants (124) faisant saillie à partir du cadre circulaire.

7. Procédé de détection d'un signal auditif par l'intermédiaire d'un ensemble microphone implantable (100) comprenant un boîtier (123, 154) ayant un diamètre de 8 mm à 10 mm positionné en dessous d'une région de peau de façon sous-cutanée chez un patient de telle sorte qu'un film polymère piézoélectrique (112, 122) fixé à un cadre circulaire (110) définissant une ouverture centrale (126) et ayant une courbure et étant positionné à l'intérieur du boîtier soit en contact vibratoire avec la région de tissu, où le boîtier est configuré pour positionner le cadre le long d'une portion supérieure du boîtier et où le cadre a un rayon de courbure de 20 mm à 25 mm de telle sorte que le film présente une surface convexe configurée pour être en contact vibratoire direct contre un tissu sous-cutané, le procédé comprenant :
la réception d'un signal auditif transmis de façon vibratoire à travers la région de peau et contre le film ;
l'actionnement du film par l'intermédiaire du signal auditif de telle sorte qu'un signal électrique représentatif du signal auditif soit produit par le film ; et
la transmission du signal électrique à une oreille interne du patient.

8. Procédé selon la revendication 7, dans lequel le film polymère piézoélectrique comprend du PVDF.

9. Procédé selon la revendication 7, dans lequel l'actionnement du film comprend en outre le traitement du signal électrique par l'intermédiaire d'un ensemble électronique (121) positionné à l'intérieur du boîtier.

10. Procédé selon la revendication 7, dans lequel l'actionnement du film comprend la communication d'une dilatation ou d'une contraction à un tampon (130) fixé à l'intérieur du boîtier et en contact avec le film.

11. Procédé selon la revendication 7, dans lequel la transmission du signal électrique comprend la transmission du signal par l'intermédiaire d'un ou de plusieurs fils (102) attachés à l'oreille interne.

12. Procédé selon la revendication 7, dans lequel la réception d'un signal auditif comprend la réception du signal par l'intermédiaire d'une portion de contact tissulaire du boîtier qui a une impédance assortie à la région de tissu.

13. Procédé selon la revendication 7, dans lequel le film polymère piézoélectrique (112, 122) est supporté par l'intermédiaire d'une pluralité de supports saillants (124) faisant saillie à partir du cadre circulaire.
